# EUROPEAN PATENT SPECIFICATION

(11) **EP 2 760 392 B1**
(45) Date of publication and mention of the grant of the patent: **30.12.2020**
(21) Application number: 12835858.7
(22) Date of filing: 28.09.2012
(51) Int. Cl.: A61F 7/00, A61F 7/12

(54) **ENDOVASCULAR COOLING CATHETER SYSTEMS WHICH EMPLOY PHASE-CHANGING HEAT EXCHANGE MEDIA**
KATHETERSYSTEME FÜR ENDOVASKULÄRE KÜHLUNG MIT PHASENWECHSELNDEN WÄRMEÜBERTRAGUNGSMEDIEN
SYSTÈMES DE CATHÉTER DE REFROIDISSEMENT ENDOVASCULAIRE UTILISANT UN MILIEU D'ÉCHANGE DE CHALEUR À CHANGEMENT DE PHASE

(30) Priority: 28.09.2011 US 201161540439 P
(43) Date of publication of application: 06.08.2014
(73) Proprietor: Zoll Circulation, Inc., San Jose, CA 95131 (US)
(72) Inventor: DABROWIAK, Jeremy, Thomas, Redwood City, CA 94065 (US); FALLER, Frederick, Burlington, MA 01803 (US)
(74) Representative: Potter Clarkson
(86) International application number: PCT/US2012/058015
(87) International publication number: WO 2013/049637

(56) References cited:
- WO-A2-2007/002946
- WO-A2-2009/117586
- US-A1- 2001 007 951
- US-A1- 2004 050 154
- US-A1- 2009 043 366
- US-A1- 2009 125 087
- US-A1- 2009 234 325
- US-A1- 2009 255 276
- US-A1- 2010 324 635
- US-A1- 2011 022 136
- US-A1- 2011 022 136
- US-A1- 2011 088 413
- US-B1- 6 301 904

## Description

### FIELD OF THE INVENTION

This invention relates generally to devices for medical treatment and more particularly to devices for endovascular heat exchange for altering or controlling body temperature in a human or animal subject.

### BACKGROUND OF THE INVENTION

Hypothermia can be induced in humans and some animals for the purpose of protecting various organs and tissues (e.g., heart, brain, kidneys) against the effects of ischemic, anoxic or toxic insult. For example, animal studies and/or clinical trials suggest that mild hypothermia can have neuroprotective and/or cardioprotective effects in animals or humans who suffer from ischemic cardiac events (e.g., myocardial infract, acute coronary syndromes, etc.), postanoxic coma after cardiopulmonary resuscitation, traumatic brain injury, stroke, subarachnoid hemorrhage, fever and neurological injury. Also, studies have shown that whole body hypothermia can ameliorate the toxic effects of radiographic contrast media on the kidneys (e.g., radiocontrast nephropathy) of patients with pre-existing renal impairment who undergo angiography procedures.

One method for inducing hypothermia is by endovascular temperature management (ETM) wherein a heat exchange catheter is inserted into a blood vessel and a thermal exchange fluid is then circulated through the heat exchange catheter. This technique can effectively cool blood flowing through the subject's vasculature and, as a result, lower the core body temperature of the subject to some desired target temperature. ETM is also capable of warming the body and/or of controlling body temperature to maintain a monitored body temperature at some selected temperature. If a controlled rate of re-warming or re-cooling from the selected target temperature is desired, that too can be accomplished by carefully controlling the amount of heat added or removed from the body and thereby controlling the temperature change of the patient.

In some situations, it is desirable to induce hypothermia rapidly. When blockage of an artery causes acute ischemia, such as is the case in acute myocardial infarction and ischemic stroke, a primary treatment objective is to reperfuse (i.e., restore blood flow to) the ischemic tissue within a short period of time (e.g., less than 5 hours) after the onset of acute clinical symptoms. Such reperfusion can be accomplished by surgery to remove or bypass the blockage or by catheter based interventions (e.g., angioplasty, stenting, atherectomy, catheter-based embolectomy, etc.) or through the use of thrombolytic drugs (e.g., tissue plasminogen activator (TPA) or streptokinase). It is currently believed that improved outcomes in such ischemic events may be achievable through the use of therapeutic hypothermia in combination with a reperfusion strategy such as surgery, catheter based intervention and/or thrombolytic drug therapy. For example, in one study, it was observed that the mean size of anterior wall myocardial infarctions is significantly reduced in patients whose core body temperature had been lowered to at least 35°C prior to reperfusion of the infarct zone. This observation is not explained by other factors including time-to-presentation, lesion location and incidence of TIMI flow prior to angioplasty. Thus, evidence exists that the ability to induce hypothermia rapidly (i.e., prior to reperfusion) may be a critical factor in optimizing patient outcomes following acute ischemic events.

The present invention provides for rapid induction of hypothermia in ETM by using a heat exchange medium that undergoes an endothermic phase change as it circulates through the heat exchange catheter.

Matter primarily exists in four phases-- solid, liquid, gas, and plasma-as well as a few other extreme phases such as critical fluids and degenerate gases. Generally, when a solid is warmed (or as pressure decreases), that solid will change to a liquid form and may eventually become a gas. For example, ice (frozen water) melts into liquid water when it is heated. As the water boils, the water evaporates and becomes water vapor. Sometimes, solids will transition directly from solid to gas, bypassing the liquid phase. This is known as sublimation.

Whenever a phase change occurs, energy is either absorbed or released. In exothermic phase changes, chemical potential energy is converted to heat energy, thereby resulting in a release of heat. In endothermic phase changes, heat energy is converted into chemical potential energy, thereby resulting in absorption of heat. Solid to liquid phase changes are typically endothermic.

The prior art has included certain heat exchange catheter systems wherein a volatile refrigerant is compressed to a liquid state, infused into a heat exchange catheter and allowed to expand within an expansion chamber, thereby undergoing an endothermic gas to liquid phase change. This gas to liquid phase change ostensibly results in absorption of heat to result in cooling of the subject's circulating blood and lowering of the subject's body temperature. Examples of heat exchange catheter systems wherein such gas-liquid phase change occurs are described in United States Patent No. 6,149,677 (Dobak III, et al.). The use of a compressed refrigerant to effect a gas to liquid phase change within an indwelling heat exchange catheter presents handling and processing issues as well as potential injury to the subject should the volatile refrigerant leak from the catheter into the subject's bloodstream. WO 2009/117586 describes devices and methods for producing two-phase coolants such as an ice slurry, and methods for using the two-phase coolants to lower the temperature or maintain a low temperature in any subject, system, object, device, or application where particular low temperatures are desired. Also described are systems for determining the cooling capacity of two-phase coolants. US 2011/0022136 describes methods and apparatus for temperature modification of a patient. The temperature modification is accomplished using an indwelling heat exchange catheter within which a fluid heat exchange medium circulates. Documents WO 2007/002946 A2 and US 7070612 B1 disclose further relevant background art.

There remains a need in the art for the development of new endovascular systems and methods for rapidly lowering a subject's body temperature in a safe and consistent manner.

### SUMMARY OF THE INVENTION

Further details, aspects, elements and attributes of the present disclosure may be appreciated by those of skill in the art after reading the detailed description and examples set forth below.

The present invention provides a body cooling system according to claim 1. Other embodiments are disclosed in the dependent claims. More generally described in this disclosure is a heat exchange catheter or other body cooling device that is insertable into or positionable in contact with the body of a human or animal subject. Such catheter or other body cooling device has an inlet, an outlet and at least one lumen through which heat exchange medium may be circulated. The catheter is connected to a source of a heat exchange medium that comprises liquid phase matter and solid phase matter, wherein the solid phase matter has a melting point not higher than about 37 degrees C and a pump or pressurization apparatus for circulating the heat exchange medium through the catheter or other body cooling device while it is inserted in or positioned on the body of a human or animal subject. During this process, at least some of the solid phase matter melts (i.e., undergoes a solid to liquid phase change), thereby removing heat from the heat exchange medium.

Also described herein, and useful for an understanding of use of the body cooling system, is a method for lowering the temperature of all or part of the body of a human or animal subject. Such method generally comprises the steps of A) positioning a heat exchange catheter or other heat exchange device in or on the subject's body; B) delivering into the catheter or other heat exchange device a flowable heat exchange medium such that it exchanges heat with the subject's body resulting in lowering of the temperature of all or part of the subject's body. The flowable heat exchange medium comprises solid phase matter that melts, either within the catheter or other heat exchange device or elsewhere within a heat exchange medium flow, path thereby removing heat from the heat exchange medium. The heat exchange medium does not directly contact or mix with any body fluid or tissue of the subject's body.

Further aspects, details, examples and embodiments of the disclosure will be appreciated by those of skill in the art upon reading the detailed description set forth below.

### DETAILED DESCRIPTION OF THE DRAWINGS

Figure 1 is a general diagram of an endovascular heat exchange system of the present disclosure comprising an extracorporeal control console and a heat exchange catheter, wherein a distal portion of the heat exchange catheter is operatively inserted into the vasculature of a human subject.
Figure 2 is a schematic diagram on a first embodiment of a solid-liquid phase changing endovascular heat exchange system of the present disclosure.
Figure 2A is a cross sectional view through line 2A-2A of Figure 2.
Figure 2B is a cross sectional view through line 2B-2B of Figure 2.
Figure 2C is a cross sectional view through line 2C-2C of Figure 2.
Figures 2D and 2E are schematic diagrams of one non-limiting example of a pump that may be used for pumping slurry in the system of Figure 2 or any other system of the present disclosure which incorporates a slurry pump.
Figure 3 is a schematic diagram of a second embodiment of a solid-liquid phase changing endovascular heat exchange system of the present disclosure.
Figure 4 is a schematic diagram of a third embodiment of a solid-liquid phase changing endovascular heat exchange system of the present disclosure.
Figure 5 is a schematic diagram of a fourth embodiment of a solid-liquid phase changing endovascular heat exchange system of the present disclosure.
Figure 5A is a schematic sectional diagram of one non-limiting example of a slurry generating device useable in the system of Figure 5 or any other system of the present disclosure which incorporates a slurry generating device.
Figure 5B is a schematic sectional diagram of a slurry generating device of Figure 5A positioned between cooling elements.
Figure 6 is a schematic diagram of a fifth embodiment of a solid-liquid phase changing endovascular heat exchange system of the present disclosure.
Figure 7 is a schematic drawing of a human subject having an alternative heat exchange catheter inserted in the subject's body.
Figure 8 is a cross sectional view of one non-limiting example of an air separator that may be useable in those embodiments of the present disclosure that have air separators.
Figure 9A is a schematic diagram of one non-limiting type of a liquid/gas/solid detector that may optionally be incorporated into any of the systems of the present disclosure to measure the relative amounts of liquid phase matter (e.g., saline solution), solid phase matter (e.g., frozen particles) and gas (e.g., air bubbles).
Figure 9B is a schematic diagram of one non-limiting type of a liquid/gas/solid detector that may optionally be incorporated into any of the systems of the present disclosure to measure the relative amounts of liquid phase matter (e.g., saline), solid phase matter (e.g., frozen particles) and gas (e.g., air bubbles).

### DETAILED DESCRIPTION

The following detailed description, the accompanying drawings and the above-set-forth brief descriptions of the drawings are intended to describe some, but not necessarily all, examples or embodiments of the disclosure. The contents of this detailed description, the accompanying drawings and the above-set-forth brief descriptions of the drawings do not limit the scope of the invention, or the scope of the following claims, in any way.

### General Aspects of a Heat Exchange Catheter System Which Uses a Heat Exchange Slurry for Rapid Cooling of a Subject's Body

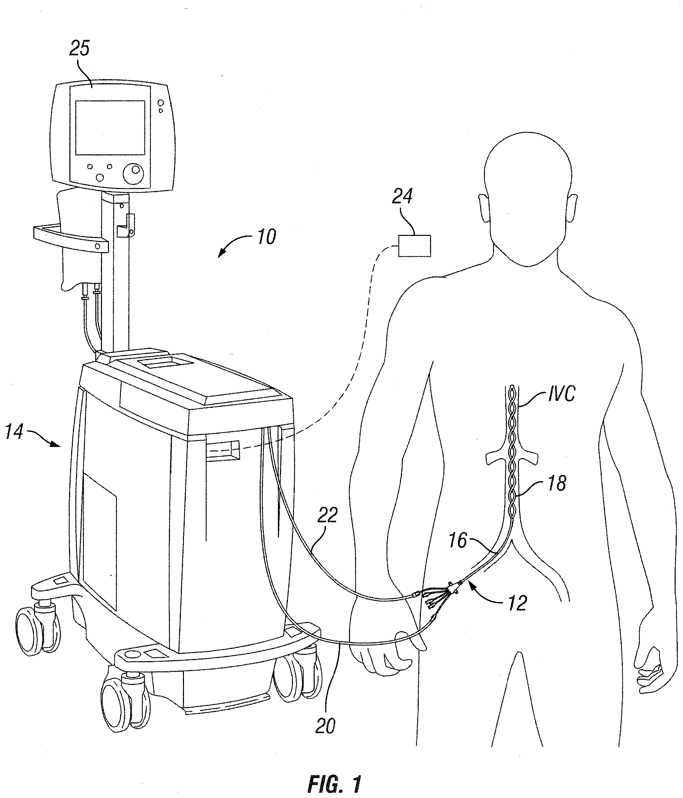

Figure 1 is a diagrammatic example of a body temperature management system 10 of the present disclosure. In this example, the body temperature management system 10 generally comprises a heat exchange catheter 12 that is connected to extracorporeal component(s) 14. A distal portion of the heat exchange catheter 16 is inserted into the vasculature of a human subject and positioned, in this example, so that a heat exchange region 18 of the catheter 16 is within the subject's inferior vena cava IVC. It is to be appreciated, however, that the catheter 16 and its heat exchange region 18 may be alternatively positioned in various other blood vessels, body lumens or body cavities, depending on the particular application and clinical setting in which the system is being utilized.

The extracorporeal component(s) 14 comprise, at minimum, a source of frozen solid/liquid heat exchange slurry and a pressure apparatus or pump for delivering that heat exchange slurry through inlet line 22 into an inflow lumen of the catheter 16. The solid-liquid heat exchange slurry then circulates through the catheter's inflow lumen and through a heat exchanger 18 on the catheter whereby the heat exchange slurry exchanges heat with blood flowing through the subject's vasculature. This exchange of heat causes some or all of the solid phase of the slurry to melt and causes cooling of the subject's flowing blood. This cooling of the subject's flowing blood results in cooling of downstream organ(s) (e.g., the heart or brain) and/or cooling of the subject's whole body. The melting of some or all of the solid particles contained in the heat exchange slurry results in substantially more cooling of the subject's flowing blood than would be attained using a liquid heat exchange medium that is devoid of solid phase particles which melt during the heat exchange. In this manner, the present disclosure cools target organ(s) or the whole body more quickly than the endovascular heat exchange systems of the prior art which utilized cooled liquid heat exchange media.

In at least some embodiments of the present invention, the heat exchange medium (including any remaining solid phase particles that have not melted) is circulated back from an outflow lumen of the catheter 16, through return line 22, and into a container within the extracorporeal system 14. In this manner, any remaining solid particles may be circulated back through the catheter 16 alone or in combination with additional heat exchange slurry or temperature controlled liquid.

### Composition and Preparation of Slurry

The heat exchange slurry may comprise any suitable mixture of frozen solid-phase particles and liquid-phase matter. Preferably, the heat exchange slurry will be sterile and sufficiently biocompatible to avoid serious injury to the subject if some or all of the slurry were to inadvertently leak from the catheter 12 into the subject's bloodstream or body. A slurry formed of frozen and liquid sodium chloride solution is one biologically compatible slurry that may be used in this disclosure. Examples of sterile saline slurries and their methods of manufacture are described in United States Patent No. 7,389,653 (Kasza et al.) entitled Medical Ice Slurry Production Device. In some applications of the present disclosure, such as those where relatively small diameter heat exchange catheters are used, it may be desirable for the solid particles of the slurry to be very small in diameter and/or for the slurry to contain a lubricious composition (e.g., a glycol such as propylene glycol) to deter agglomeration of the solid particles and/or to facilitate flow of the slurry through small diameter catheter lumens. Also, in at least some embodiments, it may be desirable for the solid particles of the slurry to be substantially spherical, or at least devoid of sharp edges, to facilitate flow of the slurry through small or tortuous catheter lumens.

Slurries useable in this disclosure may be prepared using known technology, such as a slurry ice generator having a scraped-surface vertical shell and tube heat exchanger. The inner surface of an inner tube is wiped using a wiping mechanism that comprises a sealed, rotating central shaft that has spring-loaded plastic blades or brushes extending outwardly from the shaft. Small ice crystals that form near the tube surface are wiped away from the surface by the rotating blades or brushes and are mixed with unfrozen liquid, thereby forming the slurry. Fluidized bed heat exchangers may also be used wherein steel particles circulate with the fluid to mechanically remove the crystals from the surface heat exchanger surface. The steel particles are then separated from the resultant ice slurry.

Another type of slurry generator that may be used is known as a direct contact slurry generator. In such device, an immiscible primary refrigerant is caused to evaporate in a manner that supersaturates water and forms small smooth ice crystals. However, a small amount of refrigerant may remain in ice crystals formed by this method.

Yet another type of slurry generator that may be used is known as a supercooling generator. In such device, water is pressurized and supercooled to -2°C and then released through a nozzle. As it exits the nozzle, the supercooled water changes from liquid phase to solid phase, thereby forming small ice particles.

In some embodiments, the heat exchange slurry may comprise phase change material (PCM) microcapsules disbursed in a liquid. Each PCM microcapsule comprises a core formed of phase changing material (e.g., frozen saline solution or other frozen liquid) and a shell which surrounds the core. PCM microcapsules have heretofore been reported to be useable in thermal management applications due to their ability to absorb and release large amounts of heat during phase change. Examples of PCM microcapsules and their methods of manufacture include, but are not limited to, those described in United States Patent Nos. 4,911,232 (Colvin et al.); 6,703,127 (Davis, et al.) and 6,835,334 (Davis et al.) and United States Patent Application Publication Nos. 2003/0222378 (Xing et al.); 2004/0076826 (Lee et al.); 2004/0121072 (Xing et al.); 2006/0161232 (Kasza et al.); 20080193653 (Oh et al) and 2011/0008536 (Oh, et al).

### Catheter Based Cooling of a Subject's Body Using the Solid-Liquid Heat Exchange Slurry

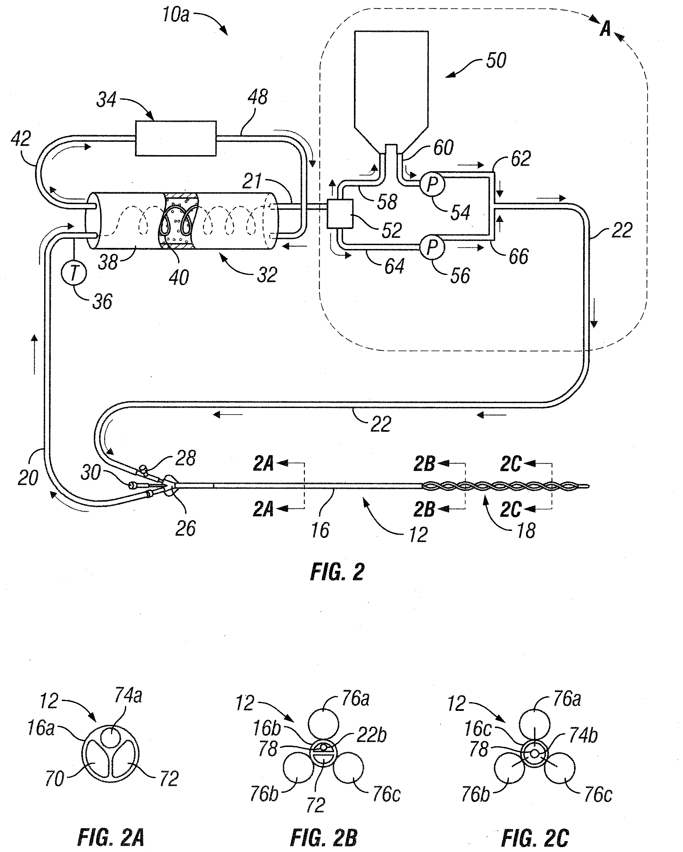

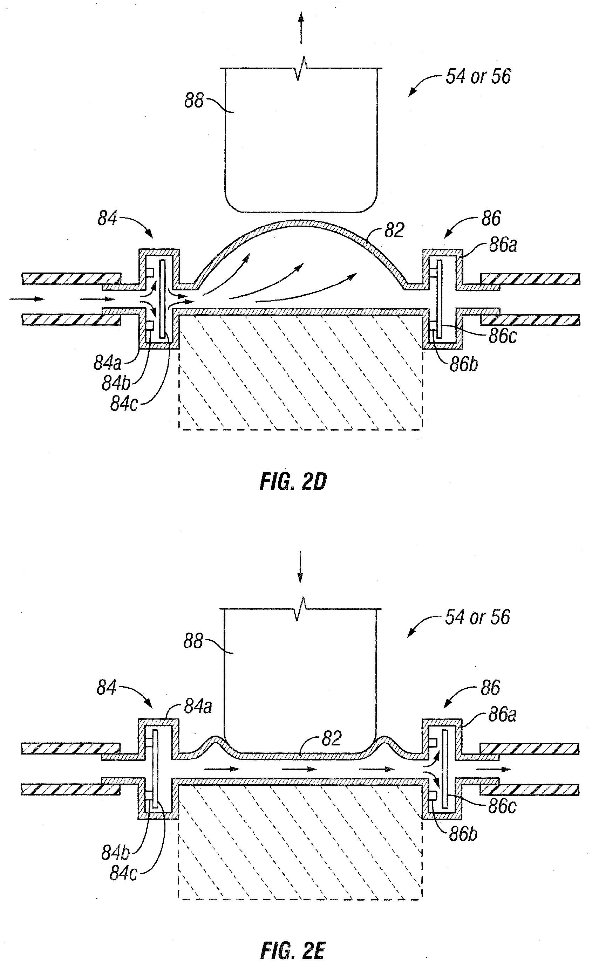

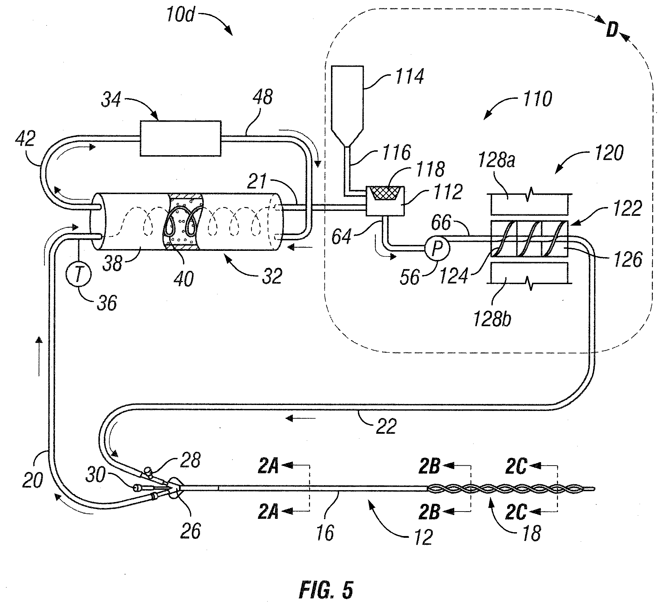

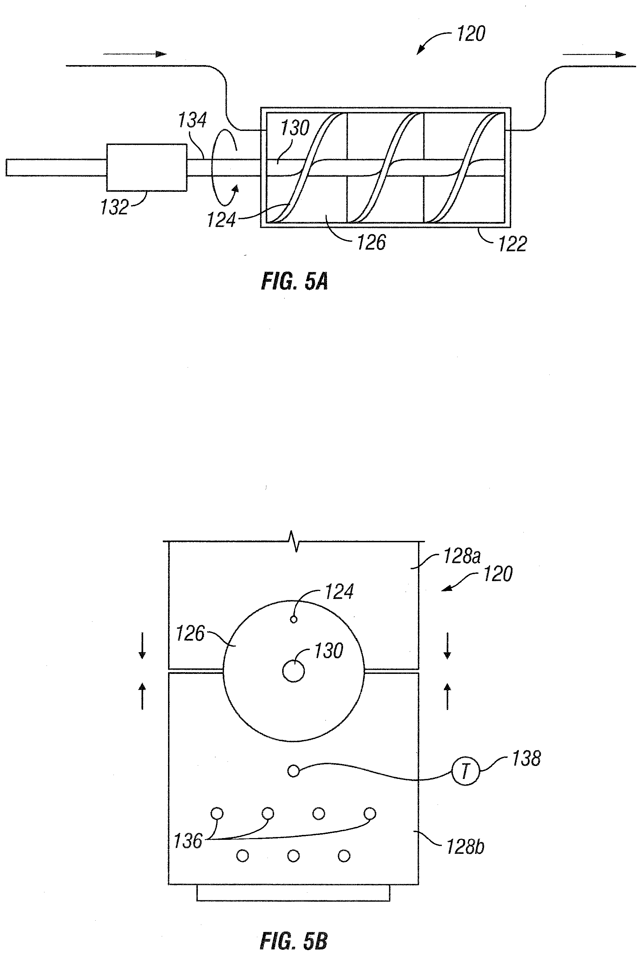

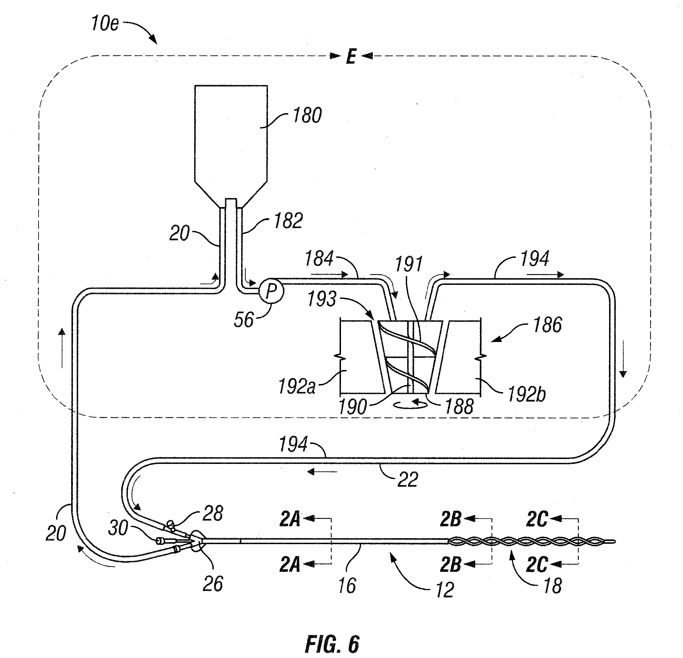

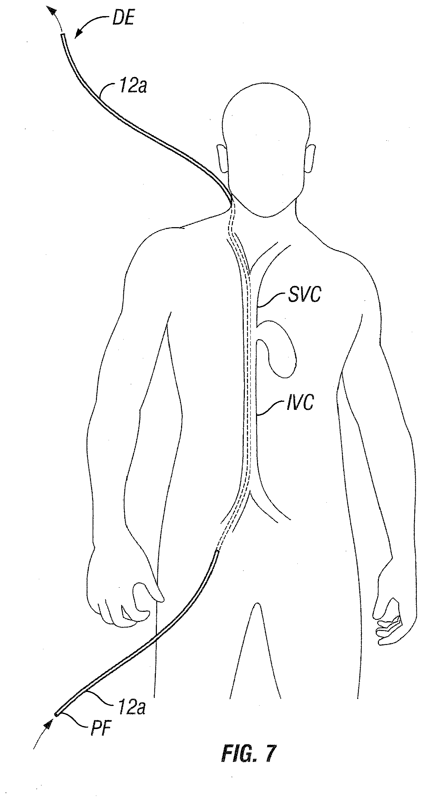

The heat exchange slurry will typically be utilized for only an initial period of time or until the temperature of the organ(s) or whole body has been lowered to a target hypothermic temperature (e.g., 34-36 degrees C). Thereafter, the system 10 may continue to control the temperature of the organ(s) or whole body by circulating a temperature controlled heat exchange liquid, such as 0.9% sodium chloride solution, through the catheter 16 in the same manner as heretofore accomplished by a number of commercially available endovascular heat exchange catheter systems, including the Thermogard XP Temperature Management System available from ZOLL Circulation of Sunnyvale, California or the InnerCool RTx™ Endovascular System available from Philips Healthcare of Andover, Massachusetts. In such embodiments, the extracorporeal system 14 may further comprise a cooler/heater for cooling or warming the heat exchange liquid, a pump for pumping that heat exchange liquid through the catheter 16, at least one temperature sensor 24 for sensing a body temperature of the subject, a user interface 25 by which a user may enter a desired target temperature and a controller, such as a computer or microprocessor, which receives the input target temperature, the sensed body temperature and, in response, controls the temperature and/or flow rate of the heat exchange liquid to attain and maintain the input target temperature. The slurry-delivering capacity may be integrated with the heat exchange fluid controlling/delivering capacity to achieve rapid initial cooling of a subject's body using the heat exchange slurry followed by maintenance of a desired target body temperature and eventual re-warming to normothermia using the traditional heat exchange liquid (e.g., saline solution). Specific examples of such systems are shown in Figures 2-7 and described herebelow. In the embodiments of Figures 2-4, a slurry concentrate is mixed with circulating heat exchange fluid to provide a slurry feed of suitable viscosity and having suitable solids content to be pumped through the particular catheter in use and to provide the desired amount of endovascular cooling. In the embodiments of Figures 5 and 6 an in-line freezer device is used to form ice particles in the circulating heat exchange fluid. Figure 7 shows an alternative catheter type that may be used in conjunction with any of the extracorporeal systems shown in Figures 1-6.

### Examples of Endovascular Temperature Management Systems Equipped to Utilize Heat Exchange Slurry for Rapid Cooling of All or Part of a Subject's Body

Figure 2 shows one example of an endovascular temperature management system 10a of the present invention. In this particular example, the heat exchange catheter 12 comprises an elongate catheter body 16 having a heat exchanger 88 mounted on a distal portion of the catheter body 12. As shown in the cross sections of Figures 2A-2C, a proximal portion 16a of the catheter body comprises a flexible shaft having an inflow lumen 70, an outflow lumen 72 and a proximal working lumen segment 74a. A mid-portion 16b of the catheter body comprises a flexible shaft having shaft having the inflow lumen 70 and working lumen 74. A distal portion 16c of the catheter body has only the working lumen 74 passing therethrough. At or near the distal end of a proximal portion 16a of the catheter body, the outflow lumen 72 terminates and communicates through openings into the proximal ends of the three generally cylindrical balloon lobes 76a, 76b and 76c. At or near the distal end of the mid-portion 16b of the catheter body, the inflow lumen 70 terminates and communicates with the distal ends of the three generally cylindrical balloon lobes 76a, 76b and 76c. The balloon lobes 76a, 76b and 76c are helically twisted, wound or otherwise helically disposed about the mid-portion 16b of the catheter body. In this example, the mid-portion 16b of the catheter body comprises a continuation or extension of the inflow lumen 70 with a smaller tube connected to and forming an extension of the working lumen 74. The attachment of the balloon lobes to the catheter may be accomplished in any appropriate manner to accomplish the circulation of heat exchange fluid described here. One such method is described in detail in United States Patent No. 6,610,083 (Keller, et al.). The distal portion 16c of the catheter body extends beyond the distal ends of the balloon lobes 76a, 76b and 76c. The tube forming the mid-region extension of the working lumen 74 continues through this distal portion 16c of the catheter body and its lumen opens through an aperture in the distal tip of the catheter 12. Thus, in this manner, a continuous working lumen that extends through the entire length of the heat exchange catheter 12. However, it is to be appreciated that as an alternative, in some embodiments, a working lumen that runs less than the entire length of the catheter may be provided to facilitate rapid exchange of guidewires and/or catheters.

As those of skill in the art will appreciate, the working lumen 74 may facilitate advancement of the catheter 12 over a guidewire and/or to facilitate infusion of fluids (e.g., saline solution, therapeutic or diagnostic substances, radiographic contrast medium, aqueous oxygen, etc.) and/or to facilitate introduction of another catheter or apparatus into the subject's body. One example of another apparatus that may be advanced through the working lumen 74 is an endovascular embodiment of the body temperature measuring apparatus 24 (e.g., a catheter or wire having a temperature sensor that is advanceable out of the distal tip of the catheter 12 and useable for sensing the temperature of the subject's flowing blood). One example of an endovascular body temperature measuring apparatus that may be advanced through working lumen 74 is the Reprieve® endovascular temperature probe manufactured by ZOLL Circulation, Inc., Sunnyvale, California.

In typical operation when the catheter 12 is inserted via a femoral vein and the heat exchanger 18 is positioned within the inferior vena cava IVC (as shown in Figure 1), the heat exchange medium (slurry or liquid) will flow distally through the inflow lumen 70, enter the distal ends of the balloon lobes 76a, 76b, 76c, flows in the proximal through the balloon lobes 76a, 76b, 76c, exit the proximal ends of the balloon lobes 76a, 76b, 76c into the outflow lumen 72 and then flow proximally trough the outflow lumen and out of the proximal end of the catheter 12.

In this system 10a, the extracorporeal components comprises an extracorporeal heat exchanger 32, a heater/cooler device 34 an air separator 52 and a a vessel 50 which contains a concentrated slurry. An outflow tube 20 connects the outflow lumen 72 the catheter 12 to an inner tube 40 of the heat exchanger 32. A temperature sensor 36 may optionally be provided to sense the temperature of heat exchange fluid returning from the catheter 12 before it enters the extracorporeal heat exchanger 32. Tubes 42 and 48 connect the heater/cooler device 34 to the shell 38 of the extracorporeal heat exchanger 32 to circulate fluid of a desired temperature through the shell 38. The outlet end of the heat exchanger tube 40 is connected to air separator 52 by way of tube 21. Thus, heat exchange fluid that returns from the catheter is passed through the heat exchanger tube 40 where its temperature is adjusted as desired and then into air separator 52. Air removed by the air separator is vented through tube 58 into the slurry-containing vessel 50 or alternatively into the atmosphere. Liquid (with any entrained ice particles) travels from the air separator 52, through tube 64 to pump 56. Slurry travels from the slurry containing vessel 50 through tube 60 to pump 54. Pumps 54 and 56 are operated to pump the desired ratio of slurry concentrate and liquid through lines 62 and 66, respectively so that they become combined in inflow line 22. This forms the desired heat exchange slurry which is delivered through inflow line 22, into inflow lumen 70 of the catheter 12 such that it circulates through the catheter 12 in the above-described manner. After all or part of the subject's body has been cooled to the desired hypothermic temperature, the slurry pump 54 may be turned off and the system will continue to operate in maintenance mode using only liquid heat exchange medium without solid ice particles.

The slurry pump 54 will be of a type that is capable of reproducibly pumping a metered volume of the relatively thick slurry concentrate. One example of such a pump is shown schematically in Figures 2D and 2E. In this non-limiting example, the pump comprises a collapsible dome diaphragm 82, an upstream check valve 84 located upstream of the dome diaphragm 82 and a downstream check valve 86 located downstream of the dome diaphragm 82. The check vales 84, 86 may be any suitable type(s) of check valves, including but not necessarily limited to ball check vales, diaphragm check valves, swing check valves, tilting disc check valves, stop check valves, lift check valves, clapper check valves, wafer check valves, duckbill check valves, electrically actuated check valves, etc. The upstream check valve 84 opens, the downstream check valve 86 closes and the dome diaphragm 82 assumes a non-compressed shape when the ram 88 cycles to its retracted position shown in Figure 2D. This causes slurry to flow through the upstream check valve 84 into the inner cavity of the dome diaphragm 82. Then, when the ram 88 cycles to its advanced position as shown in Figure 2E, the upstream check valve 84 closes, the downstream check valve 86 opens and the dome diaphragm 82 becomes compressed, thereby expelling slurry that has accumulated within the interior of the dome diaphragm 82 through the downstream check valve 86. In this manner, reciprocating motion of the ram 88 causes slurry to be pumped through the circuit. In the particular non-limiting example shown, each check valve 84, 86 comprises a rigid housing 84a, 86a which defines a flow chamber therewithin, a moveable diaphragm 84c, 86c and a seal ring 84b, 86b located on the inner wall of the housing on the upstream side of each diaphragm 84c, 86c. As shown, when the ram 88 is retracted and the dome diaphragm moves to its non-collapsed configuration, the valve diaphragms 84c and 86c are drawn inwardly, causing the upstream diaphragm 84c to separate away from the adjacent seal ring 84b (thereby opening the upstream check valve 84) and causing the downstream valve diaphragm 86c to seat firmly against the adjacent seal ring 86b (thereby closing the downstream check valve 86 to close. The speed at which the ram member 88 reciprocates will dictate the slurry concentrate throughput rate of this pump 54.

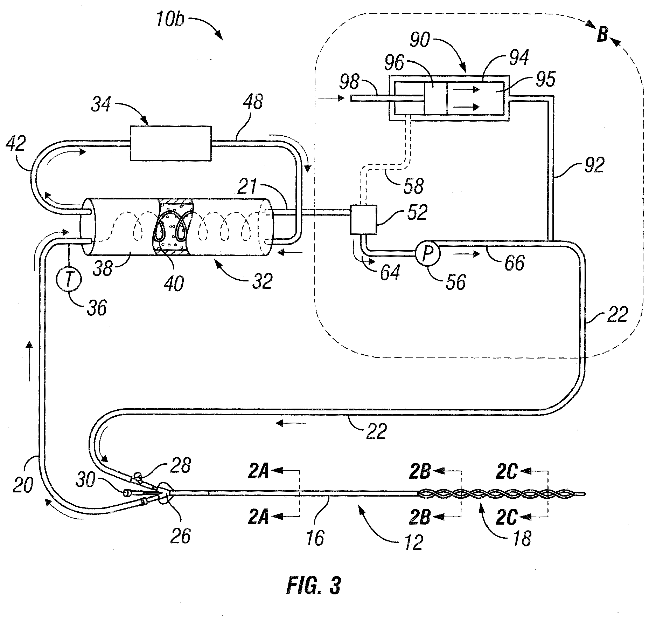

Figure 3 shows another example of an endovascular temperature management system 10b of the present invention. In this example, the system 10b incorporates a catheter 12, extracorporeal heat exchanger 32 and heater cooler 34 and interconnecting conduits 20, 22, 42, 48 of the same type as described above with respect to Figure 2. However, this system 10b differs from the showing of Figure 2 in that portion B of this system 10b replaces portion A of the system 10a seen in Figure 2.

Portion B of system 10b combines a slurry concentrate with a diluent fluid (e.g., heat exchange fluid that has returned from the catheter 12) to form a heat exchange slurry that is then circulated through the catheter 12. As shown, the outlet end of the extracorporeal heat exchanger tube 40 is connected to air separator 52 by way of tube 21. Heat exchange medium returning from the catheter 12 (with or without any remaining frozen particles) passes through the heat exchanger tube 40 where its temperature may be adjusted as desired and then into air separator 52. Air removed by the air separator 52 is vented through tube 58. The remaining heat exchange liquid (with any remaining ice particles that did not melt during the prior circulation through the circuit) is pumped by pump 56 through line 64 and into line 66. The slurry concentrate injector 90 comprises a housing 94 having an enclosed inner cavity 95 that contains a slurry concentrate, a piston 96 which is in substantially sealing contact with the inner wall of the cavity 95 and a drive 98 for driving the piston 96. As the drive 98 advances the piston 96, the piston 96 forces slurry concentrate out of the injector's inner cavity 95 through line 92. The piston drive 95 may be manual, machine-driven, hydraulic, gas-driven or may be driven in any other suitable manner that causes the piston 96 to advance at a desired rate or rates to thereby deliver desired ratio(s) of slurry concentrate to be combined with the circulating heat exchange liquid. Line 92 joins with line 66 such that the heat exchange liquid flowing through line 66 combines with slurry concentrate flowing though line 92, thereby creating heat exchange slurry of a desired consistency in inflow line 22. This heat exchange slurry then flows through inflow line 22 and circulates through the catheter 12 in the manner described above.

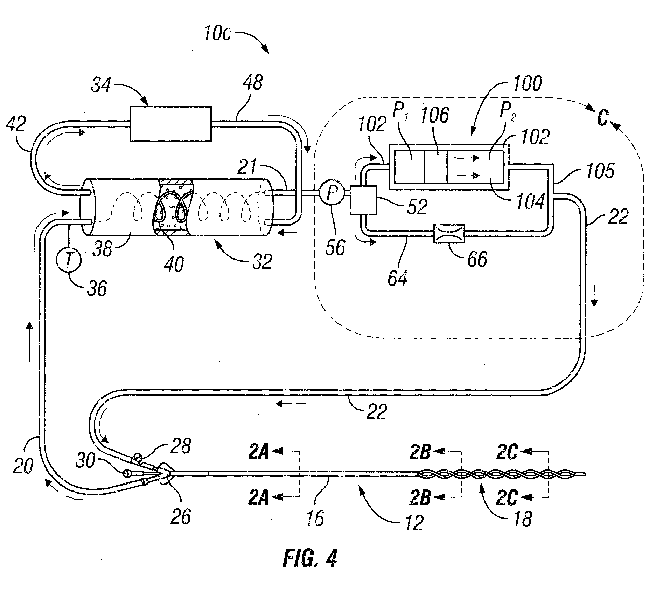

Figure 4 shows another example of an endovascular temperature management system 10b of the present invention. In this example, the system 10c incorporates a catheter 12, extracorporeal heat exchanger 32, a heater/cooler 34 and interconnecting conduits 20, 22, 42, 48 of the same type as described above with respect to Figure 2. However, this system 10c differs from the showing of Figure 2 in that portion C of this system 10c replaces portion A of the system 10a seen in Figure 2.

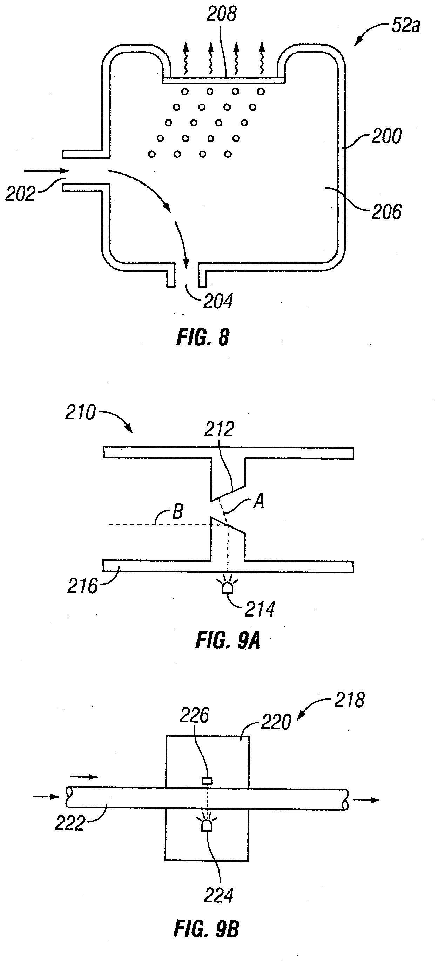

Portion C of system 10c uses a pressure driven piston system to combine a slurry concentrate with a circulating heat exchange medium (e.g., heat exchange medium that has circulated through and returned from the catheter 12) to form a desired heat exchange slurry that is then circulated through the inflow line 22 and into the catheter 12. As shown, pump 56 pumps chilled heat exchange medium (with or without any remaining frozen particles) saline through the heat exchanger outlet line 21 into air separator 52. Air removed by the air separator 52 (and potentially a quantity of overflow heat exchange medium) is vented through a vent tube 102 into a slurry concentrate injector 100 on the left side of the piston 106. The remaining heat exchange liquid (with any remaining ice particles that did not melt during the prior circulation through the circuit) flows through line 64 and through flow restrictor 66. The flow restrictor 66 may be adjustable to control the amount of back pressure in line 64 and the amount of overflow heat exchange medium that flows through vent tube 102 and into the slurry concentrate injector 100 on the left side of the piston 106. The slurry concentrate injector 100 comprises a housing 102 having an enclosed inner cavity 104 that contains a slurry concentrate on the right side of piston 106. The piston 106 is in substantially sealing slidable contact with the inner wall of the cavity 104. As air and overflow heat exchange medium passing through vent line 102 accumulates on the left side of the piston 106 the pressure P₁ on the left side of the piston will rise. When the pressure Pi on the left side of the piston 106 exceeds the pressure P₂ on the right side of the piston 106, the piston 106 will advance as indicated by arrows on Figure 4. Such advancement of the piston 106 in response to the incoming air forces slurry concentrate out of the injector's inner cavity 104 through line 105. Line 105 joins with line 64 such that the heat exchange liquid flowing through line 64 combines with slurry concentrate flowing though line 104, thereby creating heat exchange slurry of a desired consistency in inflow line 22. This heat exchange slurry then flows through inflow line 22 and circulates through the catheter 12 in the manner described above. The amount of flow restriction caused by the flow restrictor 66 may be adjusted to result in the desired heat exchange slurry consistency (i.e., the desired ratio of ice particles to liquid). In this or any embodiments of the present invention, an automated microprocessor, computer or other controller may receive signals from a sensor (see Figs. 9A and 9B) which senses the consistency of the heat exchange slurry flowing through inflow line 22. In response, such microprocessor, computer or other controller may issue control signals to other components of the system to change the relative amounts of ice particles and liquid in that heat exchange slurry. In this particular embodiment, such microprocessor, computer or other controller may be programmed to issue control signals to the fow restrictor 66 to adjust the amount of flow aloowed through the flow restrictor 66 in a manner that results in the desired heat exchange slurry consistency in line 22 (i.e., the desired ratio of ice particles to liquid).

Figure 5 shows another example of an endovascular temperature management system 10d of the present invention. In this example, the system 10d incorporates a catheter 12, extracorporeal heat exchanger 32 and heater cooler 34 and interconnecting conduits 20, 22, 42, 48 of the same type as described above with respect to Figure 2. However, this system 10d differs from the showing of Figure 2 in that portion D of this system 10d replaces portion A of the system 10a seen in Figure 2. In this system 10d, no slurry is required to be prepared offline. Instead, a fluid source, such as a bag of liquid saline 114, is spiked or otherwise connected to the system 10d and a freezer device, such as a disposable slurry generator 120, is used to form a desired amount of frozen solid matter in the circulating heat exchange liquid. In the embodiment shown, the outlet end of the extracorporeal heat exchanger tube 40 is connected to an air separator 112 by way of tube 21. Heat exchange medium returning from the catheter 12 (with or without any remaining frozen particles) passes through the heat exchanger tube 40 where its temperature may be adjusted as desired and then into air separator 112. Air removed by the air separator 112 is vented through tube a hydrophobic membrane 118. Additional heat exchange liquid (e.g., saline solution) from bag 114 passes through line 116 and into air separator 112 to combine with the remaining liquid and to make up for the volume of gas that has been vented through hydrophobic membrane 118. The heat exchange liquid (with any remaining ice particles that did not melt during the prior circulation through the circuit) is pumped by pump 56 through lines 64 and 66 and into slurry generator 120. It is to be appreciated, however, that the extracorporeal heat exchanger 32 and heater/cooler 34 are optional components of this system. If such optional components were eliminated from the system, the heat exchange medium returning from the catheter 12 via return line 20 would flow directly into line 21 and into the air separator 112.

Figures 5A and 5B show additional details of the slurry generator 120 used in this example. The slurry generator 120 comprises an enclosed generally cylindrical vessel 122 having a sealed rotating shaft 130 extending thereinto. A generally round scraper 126 is attached to the shaft 130. The rotation of the shaft may be driven by a variable speed electric motor 132 or any other suitable means. Chiller(s), such as refrigerated block members 128a, 128b having refrigeration coils 136 therein, are positioned adjacent to the vessel 122 to cool the walls of the vessel 122 to a temperature which causes the heat exchange liquid to freeze when it contacts the inner wall of the vessel 122. The refrigerated block members 128a, 128b may be reuseable and the vessel 122, shaft 130 and scraper 124 may be disposable. The refrigerated block members 128a, 128b may be moveable away from each other to allow removal and replacement of the disposable vessel 122/shaft 130/scraper 124 unit and then the refrigerated block members 128a, 128b may be moveable back toward each other to surround or abut the outer surface of the vessel 122, thereby providing for efficient heat exchange between the refrigerated block members 128a, 128b and the wall of the vessel 122. During operation, the rotating scraper 124 separates formed particles of frozen matter from the vessel wall, causing such particles of frozen matter to become combined with unfrozen liquid (and any residual frozen particles that did not melt during a prior circulation through the catheter 12) flowing through the vessel 122. This forms the desired heat exchange slurry having the desired ratio of frozen solid particles to liquid. This heat exchange slurry then exits the vessel 122 and flows through inflow line 22 and circulates through the catheter 12 in the manner described above.

The rate of ice formation in the slurry generator 120 may be controlled by adjusting the amount of cooling applied to the wall of the vessel 122 and/or the speed of the scraper 124. Temperature feedback may be used to adjust the rate of ice formation to optimize the saline return temperature and, possibly, to ensure that the amount of any ice particles remaining in the recirculated heat exchange medium is not more than can be suitably pumped by the pump 56. To facilitate this, or more temperature sensor(s) 138 may optionally be provided to sense the temperature of the refrigerated block members 128a, 128b and/or the temperature of the wall of the vessel 122. A controller may be programmed to receive the temperature(s) sensed by such temperature sensor(s) and to modify, in response, the temperature of the vessel 122 and/or the flow rate of heat exchange fluid delivered by the pump56 and/or the rate of rotation of the scraper 124, as needed, to control the amount of frozen solid phase matter in the heat exchange slurry.

Figure 6 shows another example of an endovascular temperature management system 10e of the present disclosure. In this example, the system 10e incorporates a catheter 12 and interconnecting conduits 20, 22 of the same type as described above with respect to Figure 2. However, this system 10e differs from the showing of Figure 2 in that portion E of this system 10e replaces portion A of the system 10a seen in Figure 2 and the extracorporeal heat exchanger 32 and heater cooler 34 and interconnecting conduits 42, 48 are not present, but may optionally be included. In this system 10e, no slurry is required to be prepared offline. A reservoir 180, such as a bag of liquid saline solution 180 or other container, is connected to the return line 20 which returns heat exchange medium from the catheter (including any particles of frozen solid phase matter that did not melt while circulating through the catheter 12) and to an outlet line 56. The returning heat exchange medium (including any residual frozen solid phase matter) flows from return line 20 and into reservoir 180, where it combines with any heat exchange medium that is already present in the reservoir 180. In some embodiments, the reservoir 180 may be compliant or may include an air vent to allow separation of air for the liquid (and any residual solid phase matter). Heat exchange medium then flows out of the reservoir 180 through line 182, through pump 56, through line 184 and into slurry generator 186, where a portion of the liquid phase becomes frozen to form a heat exchange slurry of a desired solid/liquid consistency.

In the system 10d of Figure 6, the slurry generator 186 comprises an enclosed generally frustoconical vessel 188 having a sealed rotating shaft 190 extending thereinto. A generally round scraper 191 of tapered diameter is attached to the shaft 190. The rotation of the shaft 188 may be driven by a variable speed electric motor (not shown) or any other suitable means. Chiller(s), such as one or more refrigerated block member(s) 192a, 192b, define a tapered cavity 193. The vessel 188, with its shaft 191 and scraper 190 positioned therein, is insertable in the tapered cavity 193, without necessarily requiring movement of the refrigerated block member(s) 192a, 192b. When the vessel 188 is positioned within the tapered cavity 193, the wall of the vessel will be in abutment with or in heat exchange proximity to the refrigerated block member(s) 192a, 192b. In this manner, the refrigerated block member(s) 192a, 192b will cool the wall of the vessel 188. During operation, the rotating scraper 191 separates formed particles of frozen matter from the wall of the vessel 188, causing such particles of frozen matter to become combined with unfrozen liquid flowing through the vessel 188. This forms the desired heat exchange slurry having the desired ratio of frozen solid particles to liquid. This heat exchange slurry then exits the vessel 188 through line 194. Line 194 is connected to the inflow line 20 through which the slurry then flows into, and circulates through, the catheter 12 in the above-described manner.

As in other embodiments, the rate of ice formation in this slurry generator 186 may be controlled by adjusting the amount of cooling applied to the wall of the vessel 188 and/or the speed of the scraper 191. Temperature feedback may be used to adjust the rate of ice formation to optimize the saline return temperature and, possibly, to ensure that the amount of any ice particles remaining in the recirculated heat exchange medium is not more than can be suitably pumped by the pump 56. To facilitate this, or more temperature sensor(s) (not shown in Figure 6) may optionally be provided ate one or more location(s) in the system 10e to sense the temperature of the refrigerated block members 128a, 128b and/or the temperature of the wall of the vessel 122 and/or the temperature of the heat exchange medium. A controller may be programmed to receive the temperature(s) sensed by such temperature sensor(s) and to modify, in response, the temperature of the vessel 188 and/or the flow rate of heat exchange fluid delivered by the pump56 and/or the rate of rotation of the scraper 124, as needed, to control the amount of frozen solid phase matter in the heat exchange slurry.

It is to be appreciated that, the catheter through which the heat exchange slurry is circulated need not be the specific catheter 12 shown and described in the above-set-forth examples, but rather, may be any suitable catheter by which heat exchange may occur between the heat exchange slurry and the subject's body or flowing blood without causing the slurry to directly contact or mix with any body fluid or tissue of the subject's body. Some catheters may be capable of use with more concentrated slurries than others because of limitations in the size or configuration of the catheter lumen(s) or other aspects of the catheter construction. Also, the heat exchange slurry need not necessarily circulate in and out of the same end of the catheter. Rather, as shown in Figure 7, in some embodiments the proximal and distal ends PE, DE of the catheter 12a may both be exteriorized while a portion of the catheter between the ends PE, DE extends through the subject's vasculature or body. In this manner, the heat exchange slurry may enter one end DE or PE of the catheter 12a and may exit the other end De, PE. Examples of various heat exchange catheters that may be used in connection with this invention include those commercially available from ZOLL Circulation, Inc. of Sunnyvale, California and Koninklijke Philips Electronics N.V. /Phillips Healthcare (InnerCool), Andover, Massachusetts .

The air separators 52 or 112 used in systems of the present invention may comprise any suitable types of air separating devices capable of removing bubbles or entrained air from liquid (and any sold or ice particles that may be present with the liquid as it passes through the air separator. Figure 8 shows a non-limiting example of one type of air separator 52a that may be used. This air separator 52a comprises a housing 200 which defines an inner chamber 206 having an inlet port 202 and an outlet port 204. An air permeable member 208 (e.g., a filter) or open vent is located at the top of the housing. Heat exchange medium or slurry flows through inlet port 204 and pools in or flows through the inner chamber 204, where entrained air will rise to the top of the chamber 206 and escape through the air permeable member 208 or open vent. The remaining liquid or slurry will flow out of the outlet port 204. In some embodiments, the air will simply vent into the environment. In other embodiments, where indicated, a vent tuve or other conduit may be attached to the opening at the top of the housing 200 so that air which flows outwardly though the air permeable member 208 or open vent will be channeled through that vent tube or conduit to a desired location (see for example the embodiment of Figure 4 where air from the air separator 52 is channeled through a vent tube 102 and used to drive a piston of an injector device 100.

Figures 9A and 9B show sensors that are used at various locations in any system of the present invention to sense the presence or absence of heat exchange medium and/or to sense the relative amounts of solid phase and liquid phase matter in slurry being circulated through the system Specifically, Figure 9A shows a level sensor 210 of a type known in the art, which may be used to sense the level of heat exchange fluid in any tank, vessel, cassette, heat exchanger or other collection chamber of the system. In this sensor 210, light leaves an emitter/detector 214 located underneath a prism that may be molded into the wall 216 of the tank, vessel, cassette, heat exchanger or other collection chamber. If the tank, vessel, cassette, heat exchanger or other collection chamber is full of liquid, light transmits through the prism (refracting slightly) along path A, reflects off of a mirror, and travels back down through the prism where it is received by the detector element of the emitter/detector 214. If the tank, vessel, cassette, heat exchanger or other collection chamber reservoir is empty, light reflects off of the 45 degree angled surface 212 of the prism along path B, thereby causing the light to be scattered around the tank, vessel, cassette, heat exchanger or other collection chamber such that substantially no light returns to the detector element of the emitter/detector 214. If the reservoir contains liquid / ice slurry, light will reflect off of the ice particles, resulting in a moderate level of transmittance to the detector element of the emitter/detector 214.

Figure 9B shows a sensor 218 that comprises a clamp 220 (off the shelf or custom) which goes around the outer diameter of a clear tube 222. Infrared light leaves an emitter 224 and enters the clear tube wall. If the tube is full of saline / water / liquid, the light transmits straight through the tube and on to the detector 226. If the tube contains air, some of the light will reflect off the tube wall, resulting in a low level of transmittance to the detector. If the tube contains a liquid / ice slurry, some of the light will reflect off of the ice particles, resulting in a moderate level of transmittance to the detector. Thus, one or more of these sensors 218 may be used to sense the amount of solid phase matter (e.g., ice) present in heat exchange fluid flowing through one or more tubes of the system.

The amount of solid phase matter in the heat exchange slurry may vary depending on the lumen size(s), heat exchanger configurations and flow restrictions inherent in design of the particular heat exchange catheter being used. Table 1 below shows examples of slurry concentrations, flow rates and resultant rates of solid phase matter delivery for several commercially available heat exchange catheters:

**Table 1**

| **Commercially Available Catheter Example** | **Flow Rate of Heat Exchange Medium Through Catheter** | **Concentration of Solid Phase Matter (e.g., Ice) in Heat Exchange Slurry** | **Resultant Rate of Delivery of Solid Phase Matter (e.g., Ice)** |
|---|---|---|---|
| Quattro® (ZOLL Circulation, Sunnyvale, CA) | 200 mL/min | 50% | 100 g/min |
| Radiant GTO® (ZOLL Circulation, Sunnyvale, CA) | 900 mL/min | 11% | 100 g/min |
| Solex/ICY® (ZOLL Circulation, Sunnyvale, CA) | 200 mL/min | 18% | 35 g/min |

Thus, for these particular heat exchange catheters, the concentration of solid phase matter within the heat exchange slurry may range from about 18% by weight to about 50% by weight and the flow rate of the heat exchange slurry through the catheter may vary from about 200 mL/min (for relatively small catheters) to about 900 mL/min (for a relatively large catheter).

Although the examples shown in the drawings and described above are specific to heat exchange catheters wherein the at least a portion of the solid phase matter melts as it circulates through the catheter, it is to be appreciated that the invention is also useable in connection with body cooling devices other than catheters (e.g., cooling blankets, pads and other surface cooling devices through which a heat exchange medium is circulated. Also, it is to be appreciated that the solid phase matter need not necessarily melt while it is within the catheter or other body cooling device. Indeed, the melting of solid phase matter anywhere within the heat exchange medium flow path-even in an extracorporeal portion of the flow path-will enhance the removal of heat from the heat exchange medium. The energy transfer (Q) through the heat exchanger is dependent on three factors:
1) the heat transfer coefficient (h) of the exchanger,
2) the amount of surface area (A) available for heat transfer, and
3) the difference in temperature (ΔT) between the heat exchange medium circulating through the heat exchanger and the blood or other body fluid circulating through the subject's body in heat exchange proximity to the heat exchanger.
This may be mathematically expressed as follows: $Q = h * A * ΔT$

Increasing the efficiency or size of the heat exchanger (h and A), generally speaking, requires increasing the cost of the heat exchanger. In embodiments where the heat exchanger is part of a sterile, disposable catheter intended for disposal after a single use, substantial increases in cost may be undesirable. Therefore, the present invention provides a more cost-effective means of increasing energy transfer through the heat exchanger by effecting a solid to liquid phase change within the heat exchange medium, thereby enhancing the removal of heat from the heat exchange medium and resulting in a greater ΔT. To effect this solid to liquid phase change, solid phase matter must be created or introduced in the heat exchange medium in a manner that does not result in clogging or fowling of the system. The examples described above avoid problems of ice clogging or fowling flow. For example, in embodiments which employ a slurry-generating device or slurry source within the extracorporeal portion of the heat exchange fluid recirculation flow path, ice particles are introduced into the flowing heat exchange fluid and at least some of those ice particles subsequently melt somewhere downstream of the location at which they were introduced. In this way, ice particles act as a heat transport mechanism, effectively increasing the surface area of the cold source and subsequently undergoing a solid to liquid phase change. This achieves greater heat transfer and more efficient and rapid cooling of the subject's body than traditional heat exchange catheter systems circulating liquid phase heat exchange fluid which cannot have an initial temperature less than 0 C.

The invention has been described hereabove with reference to certain examples or embodiments of the invention. No attempt has been made to exhaustively describe all possible embodiments and examples of the invention. Indeed, various additions, deletions, alterations and modifications may be made to the above described examples and embodiments without departing from the scope of the invention as defined in the claims.

## Claims

1. A body cooling system (10a, 10b, 10c, 10d) comprising:
a body cooling device (12) through which a heat exchange medium comprising liquid phase matter and frozen solid phase matter is recirculated, said body cooling device being selected from i) a heat exchange catheter that is insertable into the body of a human or animal subject and ii) a body surface heat exchange device that is positionable on the body of a human or animal subject, said body cooling device having an inlet, an outlet and at least one lumen; (70, 72); and
an extracorporeal system (14) which includes an extracorporeal heat exchanger (32) which connects to the inlet and outlet, said extracorporeal system (14) and at least one pump (54, 56, 88) configured to recirculate the heat exchange medium such that it flows from the extracorporeal heat exchanger (32), through the inlet of the body cooling device, through said at least one lumen, out of the outlet of the body cooling device and back into the extracorporeal heat exchanger (32),
the extracorporeal system (14) further comprising a portion (A, B, C, D, E) configured to add to the recirculating heat exchange fluid either i) a quantity of a previously prepared slurry concentrate or ii) a quantity of newly-formed frozen solid phase matter;
the body cooling system further comprising:
a sensor (218) for sensing the concentration of frozen solid particles in the heat exchange medium; and
a microprocessor, computer or other controller which receives signals from the sensor and is programmed to issue control signals to the portion (A, B, C, D or E) of the extracorporeal system (14) to thereby control the concentration of frozen solid particles in the heat exchange medium as sensed by the sensor.

2. A system according to claim 1 wherein the sensor (218) senses concentration of frozen solid phase matter in the recirculating heat exchange medium flowing into the inlet of the body cooling device.

3. A system according to claim 2 wherein the body cooling device is selected from a number of different body cooling devices which are connectable to the extracorporeal system (14) and wherein the microprocessor, computer or other controller is adapted to control the concentration of frozen solid particles such that the consistency of the heat exchange medium entering the selected body cooling device is suitable for that selected body cooling device.

4. A system according to any of claims 1-3 wherein the portion (A) of the extracorporeal system (14) is configured to add to the recirculating heat exchange fluid a quantity of a previously prepared slurry concentrate and further comprises an air separator (52) and a vessel (50) for containing the previously prepared slurry concentrate.

5. A system according to claim 1 wherein the heat exchange medium is sterile.

6. A system according to any of claims 1-3 or 5 wherein the portion (B) of the extracorporeal system (14) is configured to add to the recirculating heat exchange fluid a quantity of a previously prepared slurry concentrate and further comprises an air separator (52) and an injector (90) for injecting the previously prepared slurry concentrate into the recirculating heat exchange medium.

7. A system according to any of claims 1-3 or 5 wherein the portion (D) of the extracorporeal system (14) is configured to add to the recirculating heat exchange fluid a quantity of newly-formed frozen solid phase matter and further comprises a reservoir (114) for liquid diluent, an air separator (112) and generator (120) which comprises a vessel (122), a chiller (128a, 128b) for cooling a wall of the vessel (122) and a scraper (124) for scraping frozen solid phase matter from the wall of the vessel (122).

8. A system according to any of claims 1-3 or 5 wherein the portion (E) of the extracorporeal system (14) is configured to add to the recirculating heat exchange fluid a quantity of newly-formed frozen solid phase matter and further comprises a reservoir (180) for liquid diluent, a vessel (188) having a tapered cavity (193), a chiller (192a, 192b) for cooling a wall of the tapered cavity (193) and a scraper 191 for scraping frozen solid phase matter from the wall of the tapered cavity (193).

9. A system according to any of claims 1-3 or 5 wherein the portion (A, B, C) of the extracorporeal system (14) is configured to add a previously prepared slurry concentrate and wherein the previously prepared slurry concentrate contains greater than about 50% frozen solid phase matter.

10. A system according to any preceding claim wherein the sensed concentration of frozen solid phase matter in the heat exchange medium is controlled to be within a range of 18% to 50%.

11. A system according to claim 7 or claim 8 wherein the reservoir (114, 180) contains a liquid diluent.

12. A system according to claim 11 wherein the liquid diluent comprises a sterile solution suitable for intravenous administration.

13. A system according to claim 11 wherein the liquid diluent is selected from the group consisting of:
saline solutions;
aqueous 0.9 % NaCl solution;
water.

14. A system according to any preceding claim wherein at least some of the frozen solid phase matter melts each time the heat exchange medium recirculates through the body heat exchange device.

15. A system according to claim 14 further comprising a temperature sensor for sensing the temperature of the heat exchange medium when returning to the extracorporeal system (14) from the body cooling device (12).

16. A system according to any preceding claim wherein the extracorporeal heat exchanger alters the temperature of the recirculating heat exchange medium after it has flowed from the body heat exchange device (12) back into the extracorporeal system (14), but before it flows into the portion (A, B, C, D or E) of the extracorporeal system (14) configured to add to the recirculating heat exchange fluid either i) a quantity of a previously prepared slurry concentrate or ii) a quantity of newly-formed frozen solid phase matter.

17. A system according to claim 7 or claim 8 wherein the chiller (128a, 128b, 192a, 192b) comprises first and second chilling members that are alternately positionable in a) a spaced apart configuration that allows removal of one vessel and insertion of a new vessel to a location between the chilling member and b) a closed configuration where the chilling members are in contact with or close to at least one wall of the vessel.

18. A system according to claim 8 wherein the vessel has a frustoconical configuration and wherein the chilling apparatus has a tapered receiving area into which the vessel is insertable and from which the vessel is removable.

19. A system according to claim 1 wherein the body cooling device comprises a heat exchange catheter which comprises an elongate catheter body and a heat exchange region located on a portion of the catheter body that is insertable into the body of a subject.

20. A system according to claim 19 wherein the heat exchange region comprises a structure or material selected from:
metalized ceramic material;
a non-expandable structure;
a structure that remains at substantially the same diameter during insertion and use;
an expandable structure;
a structure that is disposable in a non-expanded configuration during insertion and subsequently expandable to an expanded configuration, wherein the expanded configuration has a greater heat exchange surface area than the non-expanded configuration;
a balloon;
a balloon structure which has a first circumscribed diameter D1 when in a nonexpanded configuration and a second circumscribed diameter D2 when in an expanded configuration; or
a material selected from; polymers, metals, ceramics, polymer-metal composites, polymer metal mixtures, ceramic-metal composites or ceramic-metal mixtures.

## Patentansprüche

1. Körperkühlsystem (10a, 10b, 10c, 10d), das Folgendes umfasst:
eine Körperkühlvorrichtung (12), über die ein Wärmeaustauschmedium, das eine Flüssigphasensubstanz und eine gefrorene Festphasensubstanz umfasst, umgewälzt wird, wobei die Körperkühlvorrichtung aus Folgenden ausgewählt ist: i) einem Wärmeaustauschkatheter, der in den Körper eines menschlichen oder tierischen Subjekts hinein einführbar ist, und ii) einer Körperoberflächenwärmeaustauschvorrichtung, die auf dem Körper eines menschlichen oder tierischen Subjekts positionierbar ist, wobei die Körperkühlvorrichtung einen Einlass, einen Auslass und wenigstens ein Lumen aufweist; (70, 72); und
ein extrakorporales System (14), das einen extrakorporalen Wärmeaustauscher (32) einschließt, der mit dem Einlass und dem Auslass verbunden ist, wobei das extrakorporale System (14) und wenigstens eine Pumpe (54, 56, 88) konfiguriert sind, um das Wärmeaustauschmedium derart umgewälzt wird, dass es von dem extrakorporalen Wärmeaustauscher (32) über den Einlass der Körperkühlvorrichtung über das wenigstens eine Lumen aus dem Auslass der Körperkühlvorrichtung und zurück in den extrakorporalen Wärmeaustauscher (32) fließt, wobei das extrakorporale System (14) ferner einen Abschnitt (A, B, C, D, E) umfasst, der konfiguriert ist, um dem umwälzenden Wärmeaustauschfluid Folgendes zuzusetzen: entweder i) eine Menge eines zuvor hergestellten Aufschlämmungskonzentrats oder ii) eine Menge einer neu ausgebildeten gefrorenen Festphasensubstanz;
wobei das Körperkühlsystem ferner Folgendes umfasst:
einen Fühler (218) zum Fühlen der Konzentration gefrorener Feststoffpartikel in dem Wärmeaustauschmedium; und
einen Mikroprozessor, Computer oder eine andere Steuervorrichtung, die Signale von dem Fühler aufnimmt und programmiert ist, um Steuersignale an den Abschnitt (A, B, C, D oder E) des extrakorporalen Systems (14) auszugeben, wobei dadurch die Konzentration gefrorener Feststoffpartikel in dem Wärmeaustauschmedium, wie durch den Fühler gefühlt, gesteuert wird.

2. System nach Anspruch 1, wobei der Fühler (218) die Konzentration einer gefrorenen Festphasensubstanz in dem umwälzenden Wärmeaustauschmedium fühlt, die in den Einlass der Körperkühlvorrichtung hinein fließt.

3. System nach Anspruch 2, wobei die Körperkühlvorrichtung aus einer Anzahl verschiedener Körperkühlvorrichtungen ausgewählt ist, die mit dem extrakorporalen System (14) verbindbar sind, und wobei der Mikroprozessor, Computer oder die andere Steuervorrichtung angepasst ist, um die Konzentration gefrorener Feststoffpartikel derart zu steuern, dass die Konsistenz des Wärmeaustauschmediums, das in die ausgewählte Körperkühlvorrichtung eintritt, für diese ausgewählte Körperkühlvorrichtung geeignet ist.

4. System nach einem der Ansprüche 1-3, wobei der Abschnitt (A) des extrakorporalen Systems (14) konfiguriert ist, um dem umwälzenden Wärmeaustauschfluid eine Menge eines zuvor hergestellten Aufschlämmungskonzentrats zuzusetzen, und ferner einen Luftabscheider (52) und ein Gefäß (50) zum Enthalten des zuvor hergestellten Aufschlämmungskonzentrats umfasst.

5. System nach Anspruch 1, wobei das Wärmeaustauschmedium steril ist.

6. System nach einem der Ansprüche 1-3 oder 5, wobei der Abschnitt (B) des extrakorporalen Systems (14) konfiguriert ist, um dem umwälzenden Wärmeaustauschfluid eine Menge eines zuvor hergestellten Aufschlämmungskonzentrats zuzusetzen, und ferner einen Luftabscheider (52) und einen Einspritzer (90) zum Einspritzen des zuvor hergestellten Aufschlämmungskonzentrats in das umwälzende Wärmeaustauschmedium hinein umfasst.

7. System nach einem der Ansprüche 1-3 oder 5, wobei der Abschnitt (D) des extrakorporalen Systems (14) konfiguriert ist, um dem umwälzenden Wärmeaustauschfluid eine Menge neu ausgebildeter gefrorener Festphasensubstanz zuzusetzen, und ferner einen Behälter (114) für ein flüssiges Verdünnungsmittel, einen Luftabscheider (112) und ein Entwicklungsgefäß (120) umfasst, der ein Gefäß (122), ein Kühlaggregat (128a, 128b) zum Kühlen einer Wand des Gefäßes (122) und einen Schaber (124) zum Schaben von gefrorenem Festphasensubstanz von der Wand des Gefäßes (122) umfasst.

8. System nach einem der Ansprüche 1-3 oder 5, wobei der Abschnitt (E) des extrakorporalen Systems (14) konfiguriert ist, um dem umwälzenden Wärmeaustauschfluid eine Menge neu ausgebildeter gefrorener Festphasensubstanz zuzusetzen, und ferner einen Behälter (180) für ein flüssiges Verdünnungsmittel, ein Gefäß (188), das einen sich verjüngenden Hohlraum (193) aufweist, ein Kühlaggregat (192a, 192b) zum Kühlen einer Wand sich verjüngenden Hohlraums (193) und einen Schaber 191 zum Schaben von gefrorenem Festphasensubstanz von der Wand des sich verjüngenden Hohlraums (193) umfasst.

9. System nach einem der Ansprüche 1-3 oder 5, wobei der Abschnitt (A, B, C) des extrakorporalen Systems (14) konfiguriert ist, um ein zuvor hergestelltes Aufschlämmungskonzentrat zuzusetzen, und wobei das zuvor hergestellte Aufschlämmungskonzentrat über etwa 50 % gefrorene Festphasensubstanz enthält.

10. System nach einem der vorhergehenden Ansprüche, wobei die gefühlte Konzentration gefrorener Festphasensubstanz in dem Wärmeaustauschmedium gesteuert ist, um innerhalb eines Bereichs von 18 % bis 50 % zu liegen.

11. System nach Anspruch 7 oder 8, wobei der Behälter (114, 180) ein flüssiges Verdünnungsmittel enthält.

12. System nach Anspruch 11, wobei das flüssige Verdünnungsmittel eine sterile Lösung umfasst, die zu einer intravenösen Verabreichung geeignet ist.

13. System nach Anspruch 11, wobei das flüssige Verdünnungsmittel aus der Gruppe ausgewählt ist, die aus Folgenden besteht:
Salzlösungen;
einer wässrigen NaCl-Lösung von 0,9 %;
Wasser.

14. System nach einem vorhergehenden Anspruch, wobei wenigstens etwas von der gefrorenen Festphasensubstanz jedes Mal schmilzt, wenn das Wärmeaustauschmedium über die Körperwärmeaustauschvorrichtung umgewälzt wird.

15. System nach Anspruch 14, das ferner einen Temperaturfühler zum Fühlen der Temperatur des Wärmeaustauschmediums umfasst, beim Zurückkehren von der Körperkühlvorrichtung (12) zu dem extrakorporalen System (14).

16. System nach einem vorhergehenden Anspruch, wobei der extrakorporale Wärmeaustauscher die Temperatur des umwälzenden Wärmeaustauschmediums ändert, nachdem es von der Körperwärmeaustauschvorrichtung (12) zurück in das extrakorporale System (14) geflossen ist, aber bevor es in den Abschnitt (A, B, C, D oder E) des extrakorporalen Systems (14) hinein fließt, das konfiguriert ist, um dem umwälzenden Wärmeaustauschfluid Folgendes zuzusetzen: entweder i) eine Menge eines zuvor hergestellten Aufschlämmungskonzentrats oder ii) eine Menge einer neu ausgebildeten gefrorenen Festphasensubstanz.

17. System nach Anspruch 7 oder 8, wobei das Kühlaggregat (128a, 128b, 192a, 192b) ein erstes und ein zweites Kühlelement umfasst, die abwechselnd in a) einer beabstandeten Konfiguration, die eine Entfernung eines Gefäßes und einen Einsatz eines neuen Gefäßes an einen Ort zwischen dem Kühlelement und b) einer geschlossenen Konfiguration positionierbar sind, an der die Kühlelemente mit wenigstens einer Wand des Gefäßes in Berührung stehen oder nah an wenigstens einer Wand des Gefäßes sind.

18. System nach Anspruch 8, wobei das Gefäß eine kegelstumpfförmige Konfiguration aufweist und wobei die Kühleinrichtung einen sich verjüngenden Aufnahmebereich aufweist, in den das Gefäß hinein einführbar ist und aus dem das Gefäß entfernbar ist.

19. System nach Anspruch 1, wobei die Körperkühlvorrichtung einen Wärmeaustauschkatheter umfasst, der einen länglichen Katheterkörper und eine Wärmeaustauschregion umfasst, die sich an einem Abschnitt des Katheterkörpers befindet, der in den Körper eines Subjekts hinein einführbar ist.

20. System nach Anspruch 19, wobei die Wärmeaustauschregion eine Struktur oder ein Material umfasst, das aus Folgenden ausgewählt ist:
einem metallisierten keramischen Werkstoff;
einer nicht erweiterbaren Struktur;
einer Struktur, die während eines Einsetzens und eines Verwendens im Wesentlichen bei dem gleichen Durchmesser bleibt;
einer erweiterbaren Struktur;
einer Struktur, die während des Einführens in einer nicht erweiterten Konfiguration wegwerfbar und anschließend zu einer erweiterten Konfiguration erweiterbar ist, wobei die erweiterte Konfiguration einen größeren Wärmeaustauschoberflächenbereich als die nicht erweiterte Konfiguration aufweist;
einem Ballon;
einer Ballonstruktur, die einen ersten umschriebenen Durchmesser D1, wenn sie sich in einer nicht erweiterten Konfiguration befindet, und einen zweiten umschriebenen Durchmesser D2 aufweist, wenn sie sich in einer erweiterten Konfiguration befindet; oder
einem Material, das aus Folgenden ausgewählt ist:
Polymeren, Metallen, Keramiken, Polymer-Metallverbundwerkstoffen, Polymer-Metallmischungen, Keramik-Metallverbundwerkstoffen oder Keramik-Metallgemischen.

## Revendications

1. Système de refroidissement corporel (10a, 10b, 10c, 10d) comprenant :
un dispositif de refroidissement corporel (12) à travers lequel un milieu d'échange thermique comprenant une matière en phase liquide et une matière en phase solide congelée est remis en circulation, ledit dispositif de refroidissement corporel étant choisi parmi i) un cathéter d'échange thermique qui peut être inséré dans le corps d'un sujet humain ou animal et ii) un dispositif d'échange thermique de surface corporelle qui peut être positionné sur le corps d'un sujet humain ou animal, ledit dispositif de refroidissement corporel ayant une entrée, une sortie et au moins une lumière ; (70, 72) ; et
un système extracorporel (14) qui comporte un échangeur thermique extracorporel (32) qui se raccorde à l'entrée et à la sortie, ledit système extracorporel (14) et au moins une pompe (54, 56, 88) conçue pour faire recirculer le milieu d'échange thermique de telle sorte qu'il s'écoule de l'échangeur thermique extracorporel (32), à travers l'entrée du dispositif de refroidissement corporel, à travers ladite au moins une lumière, hors de la sortie du dispositif de refroidissement corporel et retourne dans l'échangeur thermique extracorporel (32), le système extracorporel (14) comprenant en outre une partie (A, B, C, D, E) conçue pour ajouter au fluide d'échange thermique en recirculation soit i) une quantité d'un concentré de suspension préalablement préparé soit ii) une quantité de matière en phase solide congelée nouvellement formée ;
le système de refroidissement corporel comprenant en outre :
un capteur (218) pour détecter la concentration de particules solides congelées dans le milieu d'échange thermique ; et
un microprocesseur, ordinateur ou autre contrôleur qui reçoit des signaux du capteur et est programmé pour émettre des signaux de commande à la partie (A, B, C, D ou E) du système extracorporel (14) pour réguler ainsi la concentration de particules solides congelées dans le milieu d'échange thermique tel que détecté par le capteur.

2. Système selon la revendication 1, le capteur (218) détectant la concentration de matière en phase solide congelée dans le milieu d'échange thermique en recirculation s'écoulant dans l'entrée du dispositif de refroidissement corporel.

3. Système selon la revendication 2, le dispositif de refroidissement corporel étant choisi parmi un certain nombre de différents dispositifs de refroidissement corporel qui peuvent être raccordés au système extracorporel (14) et le microprocesseur, l'ordinateur ou un autre contrôleur étant adapté pour réguler la concentration de particules solides congelées de telle sorte que l'uniformité du milieu d'échange thermique entrant dans le dispositif de refroidissement corporel choisi est appropriée pour ce dispositif de refroidissement corporel sélectionné.

4. Système selon l'une quelconque des revendications 1 à 3, la partie (A) du système extracorporel (14) étant conçue pour ajouter au fluide d'échange thermique en recirculation une quantité d'un concentré de suspension préalablement préparé et comprenant en outre un séparateur d'air (52) et un récipient (50) pour contenir le concentré de suspension préalablement préparé.

5. Système selon la revendication 1, le milieu d'échange thermique étant stérile.

6. Système selon l'une quelconque des revendications 1 à 3 ou 5, la partie (B) du système extracorporel (14) étant conçue pour ajouter au fluide d'échange thermique en recirculation une quantité d'un concentré de suspension préalablement préparé et comprenant en outre un séparateur d'air (52) et un injecteur (90) pour injecter le concentré de suspension préalablement préparé dans le milieu d'échange thermique en recirculation.

7. Système selon l'une quelconque des revendications 1 à 3 ou 5, la partie (D) du système extracorporel (14) étant conçue pour ajouter au fluide d'échange thermique en recirculation une quantité de matière en phase solide congelée nouvellement formée et comprenant en outre un réservoir (114) pour le diluant liquide, un séparateur d'air (112) et un générateur (120) qui comprend un récipient (122), un refroidisseur (128a, 128b) pour refroidir une paroi du récipient (122) et un racloir (124) pour racler la matière en phase solide congelée de la paroi du récipient (122).

8. Système selon l'une quelconque des revendications 1 à 3 ou 5, la partie (E) du système extracorporel (14) étant conçue pour ajouter au fluide d'échange thermique en recirculation une quantité de matière en phase solide congelée nouvellement formée et comprenant en outre un réservoir (180) pour le diluant liquide, un récipient (188) ayant une cavité conique (193), un refroidisseur (192a, 192b) pour refroidir une paroi de la cavité conique (193) et un racloir 191 pour racler la matière en phase solide congelée du paroi de la cavité effilée (193).

9. Système selon l'une quelconque des revendications 1 à 3 ou 5, la partie (A, B, C) du système extracorporel (14) étant conçue pour ajouter un concentré de suspension préalablement préparé et le concentré de suspension préalablement préparé en contenant plus d'environ 50 % de matière en phase solide congelée.

10. Système selon l'une quelconque des revendications précédentes, la concentration détectée de matière en phase solide congelée dans le milieu d'échange thermique étant régulée pour se trouver à l'intérieur d'une plage de 18 % à 50 %.

11. Système selon la revendication 7 ou la revendication 8, le réservoir (114, 180) contenant un diluant liquide.

12. Système selon la revendication 11, le diluant liquide comprenant une solution stérile appropriée pour une administration intraveineuse.

13. Système selon la revendication 11, le diluant liquide étant choisi parmi le groupe constitué :
de solutions salines ;
d'une solution aqueuse de NaCl à 0,9 % ;
d'eau.

14. Système selon l'une quelconque des revendications précédentes, au moins une partie de la matière en phase solide congelée fondant à chaque fois que le milieu d'échange thermique recircule à travers le dispositif d'échange thermique corporel.

15. Système selon la revendication 14, comprenant en outre un capteur de température pour détecter la température du milieu d'échange thermique lors du retour au système extracorporel (14) depuis le dispositif de refroidissement corporel (12).

16. Système selon l'une quelconque des revendications précédentes, l'échangeur thermique extracorporel modifiant la température du milieu d'échange thermique en recirculation après qu'il s'est écoulé du dispositif d'échange thermique corporel (12) dans le système extracorporel (14), mais avant qu'il ne s'écoule dans la partie (A, B, C, D ou E) du système extracorporel (14) conçu pour ajouter au fluide d'échange thermique en recirculation soit i) une quantité d'un concentré de suspension préalablement préparé soit ii) une quantité de matière de phase solide congelée nouvellement formée.

17. Système selon la revendication 7 ou la revendication 8, le refroidisseur (128a, 128b, 192a, 192b) comprenant des premier et second éléments de refroidissement qui sont alternativement positionnables dans a) une configuration espacée qui permet le retrait d'un récipient et l'insertion d'un nouveau récipient à un emplacement entre l'élément de refroidissement et b) une configuration fermée où les éléments de refroidissement sont en contact avec ou à proximité d'au moins une paroi du récipient.

18. Système selon la revendication 8, le récipient ayant une configuration tronconique et l'appareil de refroidissement ayant une zone de réception effilée dans laquelle le récipient peut être inséré et à partir de laquelle le récipient peut être retiré.

19. Système selon la revendication 1, le dispositif de refroidissement corporel comprenant un cathéter d'échange thermique qui comprend un corps de cathéter allongé et une région d'échange thermique située sur une partie du corps de cathéter qui peut être insérée dans le corps d'un sujet.

20. Système selon la revendication 19, la région d'échange thermique comprenant une structure ou un matériau choisi parmi :
un matériau céramique métallisé ;
une structure non extensible ;
une structure qui reste sensiblement au même diamètre pendant l'insertion et l'utilisation ;
une structure extensible ;
une structure qui est jetable dans une configuration non expansée pendant l'insertion et ensuite expansible à une configuration expansée, la configuration expansée ayant une plus grande surface d'échange thermique que la configuration non expansée ;
un ballonnet ;
une structure de ballonnet qui a un premier diamètre circonscrit D1 lorsqu'il est dans une configuration non dilatée et un second diamètre circonscrit D2 lorsqu'il est dans une configuration expansée ; ou
un matériau choisi parmi ;
des polymères, des métaux, des céramiques, des composites polymère-métal, des mélanges polymère-métal, des composites céramique-métal ou des mélanges céramique-métal.
